# EUROPEAN PATENT APPLICATION

(11) **EP 0 743 321 A2**
(43) Date of publication of application: **20.11.1996**
(21) Application number: 96303478.0
(22) Date of filing: 16.05.1996
(51) Int. Cl.: C07K 14/00

(54) **Obesity gene product**

(30) Priority: 19.05.1995 US 445305
(71) Applicant: ELI LILLY AND COMPANY, Indianapolis, Indiana 46285 (US)
(72) Inventor: Hsiung, Hansen Maxwell, Carmel, Indiana 46032 (US); Smith, Dennis Paul, Indianapolis, Indiana 46254 (US)
(74) Representative: Tapping, Kenneth George

(57) **Abstract**

This invention is in the general field of molecular biology as applied to pharmaceutical or animal health research and development. The invention includes proteins, DNA compounds, vectors, and methods useful for expressing proteins that are regulate the body's volume of adipose tissue.

## Description

This invention is in the general field of molecular biology as applied to pharmaceutical or animal health research and development. The invention includes proteins, DNA compounds, vectors, and methods useful for expressing proteins that are regulate the body's volume of adipose tissue.

Physiologists have long postulated that excess fat cells laid down through overeating signals the brain that the body is obese which, in turn, causes the body to eat less and burn more fuel. G. R. Hervey, Nature 227: 629-631 (1969). Parabiotic experiments support a "feedback" model and suggest that a circulating peptide hormone may regulate the size of the body's fat depot. Furthermore, the *ob /ob* mouse model of obesity and diabetes is known to carry an autosomal recessive trait linked to a mutation in the sixth chromosome. Recently, Zhang and co-workers published the positional cloning of a mouse gene linked to this condition. Zhang et al. Nature 372: 425-32 (1994). The newly disclosed mouse *ob* gene product mentioned above is now believed to be such a hormone.

The present invention is based on the discovery and isolation of a novel obesity gene and obesity gene product. This invention is useful for producing a biologically active protein useful for regulating adipose tissue and thereby treating obesity in warm-blooded animals.

The present invention is directed to a biologically active protein of the Formula I: 
(SEQ ID NO: 1)

Val Pro Ile Xaa Xaa Val Gln Asp Asp Thr Lys Thr Leu Ile Lys Thr
1               5                   10                  15

Ile Val Thr Arg Ile Xaa Asp Ile Ser His Xaa Xaa Ser Val Ser Ser
            20                  25                  30

Lys Gln Arg Val Thr Gly Leu Asp Phe Ile Pro Gly Leu His Pro Xaa
        35                  40                  45
 
Leu Ser Leu Ser Lys Met Asp Gln Thr Leu Ala Ile Tyr Gln Gln Ile
     50                  55                  60

Leu Thr Ser Leu Pro Ser Arg Asn Val Xaa Gln Ile Ser Asn Asp Leu
 65                  70                  75                  80

Glu Asn Leu Arg Asp Leu Leu His Leu Leu Ala Xaa Ser Lys Ser Cys
                 85                  90                  95

Pro Leu Pro Gln Xaa Arg Ala Leu Glu Xaa Leu Glu Ser Leu Gly Xaa
            100                 105                 110

Val Leu Glu Ala Ser Leu Tyr Ser Thr Glu Val Val Ala Leu Ser Arg
        115                 120                 125

Leu Gln Gly Xaa Leu Gln Asp Met Leu Arg Gln Leu Asp Leu Ser Pro
    130                 135                 140

Gly Cys

145
 wherein:
Xaa at position 4 is Trp or Cys;
Xaa at position 5 is Lys or Arg;
Xaa at position 22 is Ser or Asn;
xaa at position 27 is Met or Thr;
Xaa at position 28 is Gln or absent;
Xaa at position 48 is Val or Leu;
Xaa at position 74 is Ile or Val;
Xaa at position 92 is Ser or Ala;
Xaa at position 101 is Ala or Val;
Xaa at position 106 is Thr or Ser;
Xaa at position 112 is Gly or Val; and
Xaa at position 132 is Ala or Ser.

The invention further provides nucleotide sequences that encode the protein of Formula I. Recombinant DNA vectors and host cells comprising such DNA molecules make up further embodiments of the invention. Processes for producing a protein comprising culturing such host cells and isolating proteins comprising an amino acid sequence of SEQ ID NO: 1 are also claimed. The invention further provides a method of treating obesity, which comprises administering to a warm-blooded animal in need thereof a protein of SEQ ID NO: 1. The invention further provides formulations, which comprise protein of SEQ ID NO: 1 together with one or more biologically acceptable diluents, carriers or excipients therefor.

For purposes of the present invention, as disclosed and claimed herein, the following terms and abbreviations are defined as follows:
Base pair (bp) -- refers to DNA or RNA. The abbreviations A,C,G, and T correspond to the 5'-monophosphate forms of the nucleotides (deoxy)adenine, (deoxy)cytidine, (deoxy)guanine, and (deoxy)thymine, respectively, when they occur in DNA molecules. The abbreviations U,C,G, and T correspond to the 5'-monophosphate forms of the nucleosides uracil, cytidine, guanine, and thymine, respectively when they occur in RNA molecules. In double stranded DNA, base pair may refer to a partnership of A with T or C with G. In a DNA/RNA heteroduplex, base pair may refer to a partnership of T with U or C with G.
Chelating Peptide -- An amino acid sequence capable of complexing with a multivalent metal ion.
DNA -- Deoyxribonucleic acid.
Immunoreactive Protein(s) -- a term used to collectively describe antibodies, fragments of antibodies capable of binding antigens of a similar nature as the parent antibody molecule from which they are derived, and single chain polypeptide binding molecules as described in PCT Application No. PCT/US 87/02208, International Publication No. WO 88/01649.
mRNA -- messenger RNA.
Plasmid -- an extrachromosomal self-replicating genetic element.
Reading frame -- the nucleotide sequence from which translation occurs "read" in triplets by the translational apparatus of tRNA, ribosomes and associated factors, each triplet corresponding to a particular amino acid. Because each triplet is distinct and of the same length, the coding sequence must be a multiple of three. A base pair insertion or deletion (termed a frameshift mutation) may result in two different proteins being coded for by the same DNA segment. To insure against this, the triplet codons corresponding to the desired polypeptide must be aligned in multiples of three from the initiation codon, i.e. the correct "reading frame" must be maintained. In the creation of fusion proteins containing a chelating peptide, the reading frame of the DNA sequence encoding the structural protein must be maintained in the DNA sequence encoding the chelating peptide.
Recombinant DNA Cloning Vector -- any autonomously replicating agent including, but not limited to, plasmids and phages, comprising a DNA molecule to which one or more additional DNA segments can or have been added.
Recombinant DNA Expression Vector -- any recombinant DNA cloning vector in which a promoter has been incorporated.
Replicon -- A DNA sequence that controls and allows for autonomous replication of a plasmid or other vector.
RNA -- ribonucleic acid.
Transcription -- the process whereby information contained in a nucleotide sequence of DNA is transferred to a complementary RNA sequence.
Translation -- the process whereby the genetic information of messenger RNA is used to specify and direct the synthesis of a polypeptide chain.
Treating -- describes the management and care of a human or veterinary patient for the purpose of combating a disease, condition, or disorder, of the patient. Treating includes the administration of a compound of present invention to prevent the onset of the symptoms or complications, alleviating the symptoms or complications, eliminating the disease, condition, or disorder. Treating therefor includes the inhibition of food intake, the inhibition of weight gain, and inducing weight loss in patients in need thereof.
Vector -- a replicon used for the transformation of cells in gene manipulation bearing polynucleotide sequences corresponding to appropriate protein molecules which, when combined with appropriate control sequences, confer specific properties on the host cell to be transformed. Plasmids, viruses, and bacteriophage are suitable vectors, since they are replicons in their own right. Artificial vectors are constructed by cutting and joining DNA molecules from different sources using restriction enzymes and ligases. Vectors include Recombinant DNA cloning vectors and Recombinant DNA expression vectors.

SEQ ID NO: 1 refers to the amino acid sequence set forth in the sequence listing. SEQ ID NO: 1 is following amino acid sequence. 
SEQ ID NO: 1

Val Pro Ile Xaa Xaa Val Gln Asp Asp Thr Lys Thr Leu Ile Lys Thr
1               5                   10                  15

Ile Val Thr Arg Ile Xaa Asp Ile Ser His Xaa Xaa Ser Val Ser Ser
            20                  25                  30

Lys Gln Arg Val Thr Gly Leu Asp Phe Ile Pro Gly Leu His Pro Xaa
        35                  40                  45

Leu Ser Leu Ser Lys Met Asp Gln Thr Leu Ala Ile Tyr Gln Gln Ile
    50                  55                  60

Leu Thr Ser Leu Pro Ser Arg Asn Val Xaa Gln Ile Ser Asn Asp Leu
65                  70                  75                  80

Glu Asn Leu Arg Asp Leu Leu His Leu Leu Ala Xaa Ser Lys Ser Cys
                85                  90                  95

Pro Leu Pro Gln Xaa Arg Ala Leu Glu Xaa Leu Glu Ser Leu Gly Xaa
            100                 105                 110

Val Leu Glu Ala Ser Leu Tyr Ser Thr Glu Val Val Ala Leu Ser Arg
        115                 120                 125

Leu Gln Gly Xaa Leu Gln Asp Met Leu Arg Gln Leu Asp Leu Ser Pro
    130                 135                 140

Gly Cys

145
 wherein:
Xaa at position 4 is Trp or Cys;
Xaa at position 5 is Lys or Arg;
Xaa at position 22 is Ser or Asn;
Xaa at position 27 is Met or Thr;
Xaa at position 28 is Gln or absent;
Xaa at position 48 is Val or Leu;
Xaa at position 74 is Ile or Val;
Xaa at position 92 is Ser or Ala;
Xaa at position 101 is Ala or Val;
Xaa at position 106 is Thr or Ser;
Xaa at position 112 is Gly or Val; and
Xaa at position 132 is Ala or Ser.

SEQ ID NO: 2 refers to the DNA sequence set forth in the sequence listing. SEQ ID NO: 2 is following DNA sequence.

SEQ ID NO:2

GTGCCCATCT GCAAGGTCCA GGATGACACC AAAACCCTCA TCAAGACAAT 50

TGTCACCAGG ATCAATGACA TCTCACACAC GCAGTCCGTC TCCTCCAAAC 100

AGAGGGTCAC TGGTTTGGAC TTCATCCCTG GGCTCCACCC TCTCCTGAGT 150

TTGTCCAAGA TGGACCAGAC ATTGGCGATC TACCAACAGA TCCTCACCAG 200

TCTGCCTTCC AGAAATGTGG TCCAAATATC CAATGACCTG GAGAACCTCC 250

GGGACCTTCT CCACCTGCTG GCCGCCTCCA AGAGCTGCCC CTTGCCGCAG 300

GTCAGGGCCC TGGAGAGCTT GGAGAGCTTG GGCGTTGTCC TGGAAGCTTC 350

CCTCTACTCC ACCGAGGTGG TGGCCCTGAG CCGGCTGCAG GGGTCACTAC 400

AGGACATGTT GCGGCAGCTG GACCTCAGCC CTGGGTGC 438

SEQ ID NO: 3 refers to the amino acid sequence set forth in the sequence listing. SEQ ID NO: 3 is following amino acid sequence.

SEQ ID NO: 3

Val Pro Ile Cys Lys Val Gln Asp Asp Thr Lys Thr Leu Ile Lys Thr
1               5                   10                  15

Ile Val Thr Arg Ile Asn Asp Ile Ser His Thr Gln Ser Val Ser Ser
            20                  25                  30

Lys Gln Arg Val Thr Gly Leu Asp Phe Ile Pro Gly Leu His Pro Leu
        35                  40                  45

Leu Ser Leu Ser Lys Met Asp Gln Thr Leu Ala Ile Tyr Gln Gln Ile
    50                  55                  60

Leu Thr Ser Leu Pro Ser Arg Asn Val Val Gln Ile Ser Asn Asp Leu
65                  70                  75                  80

Glu Asn Leu Arg Asp Leu Leu His Leu Leu Ala Ala Ser Lys Ser Cys
                85                  90                  95

Pro Leu Pro Gln Val Arg Ala Leu Glu Ser Leu Glu Ser Leu Gly Val
            100                 105                 110

Val Leu Glu Ala Ser Leu Tyr Ser Thr Glu Val Val Ala Leu Ser Arg
        115                 120                 125

Leu Gln Gly Ser Leu Gln Asp Met Leu Arg Gln Leu Asp Leu Ser Pro
    130                 135                 140

Gly Cys
145

SEQ ID NO: 4 refers to the DNA sequence set forth in the sequence listing. SEQ ID NO: 4 is following DNA sequence.

SEQ ID NO: 4

GTGCCCATCT GCAAGGTCCA GGATGACACC AAAACCCTCA TCAAGACAAT 50

TGTCACCAGG ATCAATGACA TCTCACACAC GTCCGTCTCC TCCAAACAGA 100

GGGTCACTGG TTTGGACTTC ATCCCTGGGC TCCACCCTCT CCTGAGTTTG 150

TCCAAGATGG ACCAGACATT GGCGATCTAC CAACAGATCC TCACCAGTCT 200

GCCTTCCAGA AATGTGGTCC AAATATCCAA TGACCTGGAG AACCTCCGGG 250

ACCTTCTCCA CCTGCTGGCC GCCTCCAAGA GCTGCCCCTT GCCGCAGGTC 300

AGGGCCCTGG AGAGCTTGGA GAGCTTGGGC GTTGTCCTGG AAGCTTCCCT 350

CTACTCCACC GAGGTGGTGG CCCTGAGCCG GCTGCAGGGG TCACTACAGG 400

ACATGTTGCG GCAGCTGGAC CTCAGCCCTG GGTGC 435

SEQ ID NO: 5 refers to the amino acid sequence set forth in the sequence listing. SEQ ID NO: 5 is following amino acid sequence.

SEQ ID NO: 5

Val Pro Ile Cys Lys Val Gln Asp Asp Thr Lys Thr Leu Ile Lys Thr
1               5                   10                  15

Ile Val Thr Arg Ile Asn Asp Ile Ser His Thr Ser Val Ser Ser Lys
            20                  25                  30

Gln Arg Val Thr Gly Leu Asp Phe Ile Pro Gly Leu His Pro Leu Leu
        35                  40                  45

Ser Leu Ser Lys Met Asp Gln Thr Leu Ala Ile Tyr Gln Gln Ile Leu
    50                  55                  60

Thr Ser Leu Pro Ser Arg Asn Val Val Gln Ile Ser Asn Asp Leu Glu
65                  70                  75                  80

Asn Leu Arg Asp Leu Leu His Leu Leu Ala Ala Ser Lys Ser Cys Pro
                85                  90                  95

Leu Pro Gln Val Arg Ala Leu Glu Ser Leu Glu Ser Leu Gly Val Val
            100                 105                 110

Leu Glu Ala Ser Leu Tyr Ser Thr Glu Val Val Ala Leu Ser Arg Leu
        115                 120                 125

Gln Gly Ser Leu Gln Asp Met Leu Arg Gln Leu Asp Leu Ser Pro Gly
    130                 135                 140

Cys
145

SEQ ID NO: 6 refers to the DNA sequence set forth in the sequence listing. SEQ ID NO: 6 is following DNA sequence.

SEQ ID NO: 6

GTGCCCATCT GGAGAGTCCA GGATGACACC AAAACCCTCA TCAAGACGAT 50

TGTCACCAGG ATCAGTGACA TTTCACACAT GCAGTCTGTC TCCTCCAAAC 100

AGAGGGTCAC CGGTTTGGAC TTCATCCCTG GGCTCCATCC TGTCCTGAGT 150

TTGTCCAAGA TGGACCAGAC CCTGGCGATC TACCAACAGA TCCTCACCAG 200

TCTGCCTTCC AGAAATGTGA TCCAAATATC GAATGACCTG GAGAACCTCC 250

GGGACCTTCT CCACCTGCTG GCCTCCTCCA AGAGCTGCCC CTTGCCCCAG 300

GCCAGGGCCC TGGAGACCTT GGAGAGCCTG GGCGGCGTCC TGGAAGCCTC 350

CCTCTACTCC ACGGAGGTGG TGGCCCTGAG CAGGCTGCAG GGGGCTCTGC 400

AGGACATGCT GCGGCAGCTG GACCTCAGCC CTGGGTGC 438

SEQ ID NO: 7 refers to the amino acid sequence set forth in the sequence listing. SEQ ID NO: 7 is following amino acid sequence.

SEQ ID NO: 7

Val Pro Ile Trp Arg Val Gln Asp Asp Thr Lys Thr Leu Ile Lys Thr
1               5                   10                  15

Ile Val Thr Arg Ile Ser Asp Ile Ser His Met Gln Ser Val Ser Ser
            20                  25                  30

Lys Gln Arg Val Thr Gly Leu Asp Phe Ile Pro Gly Leu His Pro Val
        35                  40                  45

Leu Ser Leu Ser Lys Met Asp Gln Thr Leu Ala Ile Tyr Gln Gln Ile
    50                  55                  60

Leu Thr Ser Leu Pro Ser Arg Asn Val Ile Gln Ile Ser Asn Asp Leu
65                  70                  75                  80

Glu Asn Leu Arg Asp Leu Leu His Leu Leu Ala Ser Ser Lys Ser Cys
                85                  90                  95

Pro Leu Pro Gln Ala Arg Ala Leu Glu Thr Leu Glu Ser Leu Gly Gly
            100                 105                 110

Val Leu Glu Ala Ser Leu Tyr Ser Thr Glu Val Val Ala Leu Ser Arg
        115                 120                 125

Leu Gln Gly Ala Leu Gln Asp Met Leu Arg Gln Leu Asp Leu Ser Pro
    130                 135                 140

Gly Cys
145

The nucleotide and amino acid abbreviations are accepted by the United States Patent and Trademark Office as set forth in 37 C.F.R. § 1.822 (b) (2) (1993). One skilled in the art would recognize that certain amino acids are prone to rearrangement. For example, Asp may rearrange to aspartimide and isoasparigine as described in I. Schön et al., Int. J. Peptide Protein Res. 14: 485-94 (1979) and references cited therein. These rearrangement derivatives are included within the scope of the present invention. Unless otherwise indicated the amino acids are in the L configuration.

As noted above the present invention provides a protein of Formula I. The preferred proteins of the present invention are those represented by SEQ ID NO: 3, SEQ ID NO: 5, and SEQ ID NO: 7.

The invention further provides DNA compounds that encode for the protein of SEQ ID NO: 1. The preferred DNA compounds are those represented by SEQ ID NO: 2, SEQ ID NO: 4, and SEQ ID NO: 6.

The proteins of the present invention may be produced either by recombinant DNA technology or well known chemical procedures, such as solution or solid-phase peptide synthesis, or semi-synthesis in solution beginning with protein fragments coupled through conventional solution methods.

The principles of solid phase chemical synthesis of polypeptides are well known in the art and may be found in general texts in the area such as Dugas, H. and Penney, C., Bioorganic Chemistry Springer-Verlag, New York, pgs. 54-92 (1981). For example, peptides may be synthesized by solid-phase methodology utilizing an PE-Applied Biosystems 430A peptide synthesizer (commercially available from Applied Biosystems, Foster City California) and synthesis cycles supplied by Applied Biosystems. Boc amino acids and other reagents are commercially available from PE-Applied Biosystems and other chemical supply houses. Sequential Boc chemistry using double couple protocols are applied to the starting p-methyl benzhydryl amine resins for the production of C-terminal carboxamides. For the production of C-terminal acids, the corresponding PAM resin is used. Arginine, Asparagine, Glutamine, Histidine and Methionine are coupled using performed hydroxy benzotriazole esters. The following side chain protection may be used:
Arg, Tosyl
Asp, cyclohexyl or benzyl
Cys, 4-methylbenzyl
Glu, cyclohexyl
His, benzyloxymethyl
Lys, 2-chlorobenzyloxycarbonyl
Met, sulfoxide
Ser, Benzyl
Thr, Benzyl
Trp, formyl
Tyr, 4-bromo carbobenzoxy
Boc deprotection may be accomplished with trifluoroacetic acid (TFA) in methylene chloride. Formyl removal from Trp is accomplished by treatment of the peptidyl resin with 20% piperidine in dimethylformamide for 60 minutes at 4°C. Met(O) can be reduced by treatment of the peptidyl resin with TFA/dimethylsulfide/conHCl (95/5/1) at 25°C for 60 minutes. Following the above pre-treatments, the peptides may be further deprotected and cleaved from the resin with anhydrous hydrogen fluoride containing a mixture of 10% m-cresol or m-cresol/10% p-thiocresol or m-cresol/p-thiocresol/ dimethylsulfide. Cleavage of the side chain protecting group(s) and of the peptide from the resin is carried out at zero degrees Centigrade or below, preferably -20°C for thirty minutes followed by thirty minutes at 0°C. After removal of the HF, the peptide/resin is washed with ether. The peptide is extracted with glacial acetic acid and lyophilized. Purification is accomplished by reverse-phase C18 chromatography (Vydac) column in .1% TFA with a gradient of increasing acetonitrile concentration. One skilled in the art recognizes that the solid phase synthesis could also be accomplished using the FMOC strategy and a TFA/scavenger cleavage mixture.

Preferably, the proteins are prepared using recombinant techniques. In general the proteins are prepared from a precursor protein comprising the protein by
(a) culturing a host cell;
(b) optionally processing the precursor protein to produce the protein; and
(c) isolating the protein.

The claimed DNA sequences are useful for expressing the *ob* gene product either by direct expression or as fusion or precursor protein. When the claimed sequences are used in a fusion gene, the resulting product will require enzymatic or chemical cleavage. A variety of peptidases which cleave a polypeptide at specific sites or digest the peptides from the amino or carboxy termini (e.g. diaminopeptidase) of the peptide chain are known. Furthermore, particular chemicals (e.g. cyanogen bromide) will cleave a polypeptide chain at specific sites. The skilled artisan will appreciate the modifications necessary to the amino acid sequence (and synthetic or semi-synthetic coding sequence if recombinant means are employed) to incorporate site-specific internal cleavage sites. See U.S. Patent No. 5,126,249; Carter P., Site Specific Proteolysis of Fusion Proteins, Ch. 13 in Protein Purification: From Molecular Mechanisms to Large Scale Processes, American Chemical Soc., Washington, D.C. (1990).

Construction of suitable vectors containing the desired coding and control sequences employ standard ligation techniques. Isolated plasmids or DNA fragments are cleaved, tailored, and religated in the form desired to form the plasmids required.

To effect the translation of the desired protein, one inserts the engineered synthetic DNA sequence in any of a plethora of appropriate recombinant DNA expression vectors through the use of appropriate restriction endonucleases. A synthetic coding sequence is designed to possess restriction endonuclease cleavage sites at either end of the transcript to facilitate isolation from and integration into these expression and amplification and expression plasmids. The isolated cDNA coding sequence may be readily modified by the use of synthetic linkers to facilitate the incorporation of this sequence into the desired cloning vectors by techniques well known in the art. The particular endonucleases employed will be dictated by the restriction endonuclease cleavage pattern of the parent expression vector to be employed. The restriction sites are chosen so as to properly orient the coding sequence with control sequences to achieve proper in-frame reading and expression of the claimed protein.

In general, plasmid vectors containing promoters and control sequences that are derived from species compatible with the host cell are used with these hosts. The vector ordinarily carries a replication origin as well as marker sequences that are capable of providing phenotypic selection in transformed cells. For example, E. coli is typically transformed using pBR322, a plasmid derived from an E. coli species (Bolivar, et al., Gene 2: 95 (1977)). Plasmid pBR322 contains genes for ampicillin and tetracycline resistance and thus provides easy means for identifying transformed cells. The pBR322 plasmid, or other microbial plasmid must also contain or be modified to contain promoters and other control elements commonly used in recombinant DNA technology.

The desired coding sequence is inserted into an expression vector in the proper orientation to be transcribed from a promoter and ribosome binding site, both of which should be functional in the host cell in which the protein is to be expressed. An example of such an expression vector is a plasmid described in Belagaje et al., U.S. patent No. 5,304,493, the teachings of which are herein incorporated by reference. The gene encoding A-C-B proinsulin described in U.S. patent No. 5,304,493 can be removed from the plasmid pRB182 with restriction enzymes NdeI and BamHI. The claimed DNA sequences of the present invention can be inserted into the plasmid backbone on a NdeI/BamHI restriction fragment cassette.

In general, procaryotes are used for cloning of DNA sequences in constructing the vectors useful in the invention. For example, E. coli K12 strain 294 (ATCC No. 31446) is particularly useful. Other microbial strains which may be used include E. coli B and E. coli X1776 (ATCC No. 31537). These examples are illustrative rather than limiting.

Procaryotes also are used for expression. The aforementioned strains, as well as E. coli W3110 (prototrophic, ATCC No. 27325), bacilli such as Bacillus subtilis, and other enterobacteriaceae such as Salmonella typhimurium or Serratia marcescans, and various pseudomonas species may be used. Promoters suitable for use with prokaryotic hosts include the b-lactamase (vector pGX2907 [ATCC 39344] contains the replicon and b-lactamase gene) and lactose promoter systems (Chang et al., Nature, 275:615 (1978); and Goeddel et al., Nature 281:544 (1979)), alkaline phosphatase, the tryptophan (trp) promoter system (vector pATH1 [ATCC 37695] is designed to facilitate expression of an open reading frame as a trpE fusion protein under control of the trp promoter) and hybrid promoters such as the tac promoter (isolatable from plasmid pDR540 ATCC-37282). However, other functional bacterial promoters, whose nucleotide sequences are generally known, enable one of skill in the art to ligate them to DNA encoding the protein using linkers or adaptors to supply any required restriction sites. Promoters for use in bacterial systems also will contain a Shine-Dalgarno sequence operably linked to the DNA encoding protein.

The claimed DNA molecules may also be recombinantly produced in eukaryotic expression systems. Preferred promoters controlling transcription in mammalian host cells may be obtained from various sources, for example, the genomes of viruses such as: polyoma, Simian Virus 40 (SV40), adenovirus, retroviruses, hepatitis-B virus and most preferably cytomegalovirus, or from heterologous mammalian promoters, e.g. b-actin promoter. The early and late promoters of the SV40 virus are conveniently obtained as an SV40 restriction fragment which also contains the SV40 viral origin of replication. Fiers, et al., Nature, 273:113 (1978). The entire SV40 genome may be obtained from plasmid pBRSV, ATCC 45019. The immediate early promoter of the human cytomegalovirus may be obtained from plasmid pCMBb (ATCC 77177). Of course, promoters from the host cell or related species also are useful herein.

Transcription of the claimed DNA by higher eucaryotes is increased by inserting an enhancer sequence into the vector. Enhancers are cis-acting elements of DNA, usually about 10-300 bp, that act on a promoter to increase its transcription. Enhancers are relatively oriented and positioned independently and have been found 5'(Laimins, L. et al., PNAS 78:993 (1981)) and 3' (Lusky, M. L., et al., Mol. Cell Bio. 3:1108 (1983)) to the transcription unit, within an intron (Banerji, J. L. et al., Cell 33:729 (1983)) as well as within the coding sequence itself (Osborne, T. F., et al., Mol. Cell Bio. 4:1293 (1984)). Many enhancer sequences are now known from mammalian genes (globin, RSV, SV40, EMC, elastase, albumin, a-fetoprotein and insulin). Typically, however, one will use an enhancer from a eukaryotic cell virus. Examples include the SV40 late enhancer, the cytomegalovirus early promoter enhancer, the polyoma enhancer on the late side of the replication origin, and adenovirus enhancers.

Expression vectors used in eukaryotic host cells (yeast, fungi, insect, plant, animal, human or nucleated cells from other multicellular organisms) will also contain sequences necessary for the termination of transcription which may affect mRNA expression. These regions are transcribed as polyadenylated segments in the untranslated portion of the mRNA encoding protein. The 3' untranslated regions also include transcription termination sites.

Expression vectors may contain a selection gene, also termed a selectable marker. Examples of suitable selectable markers for mammalian cells are dihydrofolate reductase (DHFR, which may be derived from the BglII/HindIII restriction fragment of pJOD-10 [ATCC 68815]), thymidine kinase (herpes simplex virus thymidine kinase is contained on the BamHI fragment of vP-5 clone [ATCC 2028]) or neomycin (G418) resistance genes (obtainable from pNN414 yeast artificial chromosome vector [ATCC 37682]). When such selectable markers are successfully transferred into a mammalian host cell, the transfected mammalian host cell can survive if placed under selective pressure. There are two widely used distinct categories of selective regimes. The first category is based on a cell's metabolism and the use of a mutant cell line that lacks the ability to grow without a supplemented media. Two examples are: CHO DHFR⁻ cells (ATCC CRL-9096) and mouse LTK⁻ cells (L-M(TK-) ATCC CCL-2.3). These cells lack the ability to grow without the addition of such nutrients as thymidine or hypoxanthine. Because these cells lack certain genes necessary for a complete nucleotide synthesis pathway, they cannot survive unless the missing nucleotides are provided in a supplemented media. An alternative to supplementing the media is to introduce an intact DHFR or TK gene into cells lacking the respective genes, thus altering their growth requirements. Individual cells, which were not transformed with the DHFR or TK gene, will not be capable of survival in nonsupplemented media.

The second category is dominant selection that refers to a selection scheme used in any cell type and does not require the use of a mutant cell line. These schemes typically use a drug to arrest growth of a host cell. Those cells, which have a novel gene, would express a protein conveying drug resistance and would survive the selection. Examples of such dominant selection use the drugs neomycin, Southern P. and Berg, P., J. Molec. Appl. Genet. 1: 327 (1982), mycophenolic acid, Mulligan, R. C. and Berg, P. Science 209:1422 (1980), or hygromycin, Sugden, B. et al., Mol Cell. Biol. 5:410-413 (1985). The three examples given above employ bacterial genes under eukaryotic control to convey resistance to the appropriate drug G418 or neomycin (geneticin), xgpt (mycophenolic acid) or hygromycin, respectively.

A preferred vector for eucaryotic expression is pRc/CMV. pRc/CMV is commercially available from Invitrogen Corporation, 3985 Sorrento Valley Blvd., San Diego, CA 92121. To confirm correct sequences in plasmids constructed, the ligation mixtures are used to transform E. coli K12 strain DH10B (ATCC 31446) and successful transformants selected by antibiotic resistance where appropriate. Plasmids from the transformants are prepared, analyzed by restriction and/or sequence by the method of Messing, et al., Nucleic Acids Res. 9:309 (1981).

Host cells may be transformed with the expression vectors of this invention and cultured in conventional nutrient media modified as is appropriate for inducing promoters, selecting transformants or amplifying genes. The culture conditions, such as temperature, pH and the like, are those previously used with the host cell selected for expression, and will be apparent to the ordinarily skilled artisan. The techniques of transforming cells with the aforementioned vectors are well known in the art and may be found in such general references as Maniatis, et al., Molecular Cloning: A Laboratory Manual, Cold Spring Harbor Press, Cold Spring Harbor Laboratory, Cold Spring Harbor, New York (1989), or Current Protocols in Molecular Biology (1989) and supplements.

Preferred suitable host cells for expressing the vectors encoding the claimed proteins in higher eucaryotes include: African green monkey kidney line cell line transformed by SV40 (COS-7, ATCC CRL-1651); transformed human primary embryonal kidney cell line 293, (Graham, F. L. et al., J. Gen Virol. 36:59-72 (1977), Virology 77:319-329, Virology 86:10-21); baby hamster kidney cells (BHK-21(C-13), ATCC CCL-10, Virology 16:147 (1962)); chinese hamster ovary cells CHO-DHFR⁻ (ATCC CRL-9096), mouse Sertoli cells (TM4, ATCC CRL-1715, Biol. Reprod. 23:243-250 (1980)); african green monkey kidney cells (VERO 76, ATCC CRL-1587); human cervical epitheloid carcinoma cells (HeLa, ATCC CCL-2); canine kidney cells (MDCK, ATCC CCL-34); buffalo rat liver cells (BRL 3A, ATCC CRL-1442); human diploid lung cells (WI-38, ATCC CCL-75); human hepatocellular carcinoma cells (Hep G2, ATCC HB-8065);and mouse mammary tumor cells (MMT 060562, ATCC CCL51).

In addition to prokaryotes, unicellular eukaryotes such as yeast cultures may also be used. Saccharomyces cerevisiae, or common baker's yeast is the most commonly used eukaryotic microorganism, although a number of other strains are commonly available. For expression in Saccharomyces, the plasmid YRp7, for example, (ATCC-40053, Stinchcomb, et al., Nature 282:39 (1979); Kingsman et al., Gene 7:141 (1979); Tschemper et al., Gene 10:157 (1980)) is commonly used. This plasmid already contains the trp gene which provides a selection marker for a mutant strain of yeast lacking the ability to grow in tryptophan, for example ATCC no. 44076 or PEP4-1 (Jones, Genetics 85:12 (1977)).

Suitable promoting sequences for use with yeast hosts include the promoters for 3-phosphoglycerate kinase (found on plasmid pAP12BD ATCC 53231 and described in U.S. Patent No. 4,935,350, June 19, 1990) or other glycolytic enzymes such as enolase (found on plasmid pAC1 ATCC 39532), glyceraldehyde-3-phosphate dehydrogenase (derived from plasmid pHcGAPC1 ATCC 57090, 57091), zymomonas mobilis (United States Patent No. 5,000,000 issued March 19, 1991), hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which contain inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein (contained on plasmid vector pCL28XhoLHBPV ATCC 39475, United States Patent No. 4,840,896), glyceraldehyde 3-phosphate dehydrogenase, and enzymes responsible for maltose and galactose (GAL1 found on plasmid pRY121 ATCC 37658) utilization. Suitable vectors and promoters for use in yeast expression are further described in R. Hitzeman et al., European Patent Publication No. 73,657A. Yeast enhancers such as the UAS Gal from Saccharomyces cerevisiae (found in conjunction with the CYC1 promoter on plasmid YEpsec--hIlbeta ATCC 67024), also are advantageously used with yeast promoters.

The following examples will help describe how the invention is practiced and will illustrate the characteristics of the claimed DNA molecules, vectors, host cells, and methods of the invention. The scope of the present invention is not to be construed as merely consisting of the following examples.

### Example 1

### Porcine OB Gene and Gene Product

Total RNA was isolated from porcine fat tissue obtained from Pel-Freez®, Pel-Freez Inc., and the cDNA was cloned in accordance with the techniques described in Hsiung et al., Neuropeptide 25: 1-10 (1994).

Primers were designed based on the published amino acid sequence of the human *ob* gene. The primers were prepared for use in polymerase chain reaction (PCR) amplification methods using a Model 380A DNA synthesizers (PE-Applied Biosystems, Inc., 850 Lincoln Center Drive, Foster City, CA 94404). Primers PCROB-1 (12504) (ATG CAT TGG GGA MCC CTG TG) (SEQ ID NO: 8), PCROB-2 (12505) (GG ATT CTT GTG GCT TTG GYC CTA TCT) (SEQ ID NO: 9), PCROB-3 (12506) (TCA GCA CCC AGG GCT GAG GTC CA) (SEQ ID NO: 10), and PCROB-4 (12507) (CAT GTC CTG CAG AGA CCC CTG CAG CCT GCT CA) (SEQ ID NO: 11) were prepared.

For cDNA synthesis, total RNA 1 µL (1 µg/µl) isolated from porcine adipose tissue and 1 µL Perkin Elmer Random primers (50 µM) in a total volume of 12 µL were annealed for 10 minutes at 70°C and then cooled on ice. The following were then added to the annealed mixture: 4 µL of BRL 5x H-reverse transcriptase (RT) reaction buffer (Gibco-BRL CAT#28025-013), 2 µL of 0.1 M DTT, 1 µL of 10 mM dNTPs. This annealed mixture was then incubated at 37°C for 2 minutes before adding 1 µL BRL M-MLV-reverse transcriptase (200 U/µL) (CAT#28025-013) and incubated at 37°C for additional 1 hour. After incubation the mixture was heated at 95°C for 5 minutes and then was cooled on ice.

For amplification of cDNA, the polymerase chain reaction (PCR) was carried out in a reaction mixture (100 µL) containing the above cDNA reaction mixture (1 µL), 2.5 units of AmpliTaq DNA polymerase (Perkin-Elmer Corporation) 10 µl of 10x PCR reaction buffer (Perkin-Elmer Corporation), 4 µL of 10 mM dNTPs and 50 pmol each of the sense (PCROB-1) and antisense (PCROB-3) primers for porcine OB amplification. The condition for PCR was 95°C for 1 minute, 57°C for 1 minute and 72°C for 1 minute for 30 cycles using a PCR DNA Thermal Cycler (Perkin-Elmer Corporation). After PCR amplification, 5 µL BRL T4 DNA polymerase (5 U/µL), 2 µL BRL T4 polynucleotide kinase (10 U/µL), and 5 µL ATP (10 mM) were added to the PCR reaction mixture (100 µl) directly and incubated for 30 minutes at 37°C. After the incubation the reaction mixture was heated at 95°C for 5 minutes and then was cooled on ice. The 500 bp fragment (∼0.5 µg) was purified by agarose gel electrophoresis and isolated by the freeze-squeeze method. The 500 bp fragment (∼0.2 µg) was then ligated into SmaI linearized pUC18 plasmid (∼1 µg) and the ligation mixture was used to transform DH5α (BRL) competent cells. The transformation mixture was plated on 0.02% X-Gal TY broth plates containing ampicillin (Amp) (100 µg/mL) and was then incubated overnight at 37°C. White clones were picked and were grown at 37°C overnight in TY broth containing Amp (100 µg/mL). The plasmid was isolated using a Wizard Miniprep DNA purification system (Promega) and submitted for DNA seqeuncing on a Applied Biosystem 370 DNA sequencer. The sequence obtained is defined herein as SEQ ID NO: 6.

### Example 2

### Bovine OB Gene and Gene Product

The DNA sequence of SEQ ID NO: 2 was obtained by techniques analogous to Example 1, except sense (PCROB-2) and antisense (PCROB-3) primers were used for bovine OB cDNA amplification.

### Example 3

### Bovine OB Gene and Gene Product

The DNA sequence of SEQ ID NO: 4 was obtained by techniques analogous to Example 1, except sense (PCROB-2) and antisense (PCROB-3) primers were used for bovine OB cDNA amplification.

### Example 4

### Vector Construction

A plasmid containing the DNA sequence encoding the claimed protein is constructed to include NdeI and BamHI restriction sites. The plasmid carrying the cloned PCR product is digested with NdeI and BamHI restriction enzymes. The small - 450bp fragment is gel-purified and ligated into the vector pRB182 from which the coding sequence for A-C-B proinsulin is deleted. The ligation products are transformed into E. coli DH10B (commercially available from GIBCO-BRL) and colonies growing on tryptone-yeast (DIFCO) plates supplemented with 10 mg/mL of tetracycline are analyzed. Plasmid DNA is isolated, digested with NdeI and BamHI and the resulting fragments are separated by agarose gel electrophoresis. Plasmids containing the expected - 450bp NdeI to BamHI fragment are kept. E. coli K12 RV308 (available from the NRRL under deposit number B-15624) are transformed with this second plasmid, resulting in a culture suitable for expressing the protein.

The techniques of transforming cells with the aforementioned vectors are well known in the art and may be found in such general references as Maniatis, et al. (1988) Molecular Cloning: A Laboratory Manual, Cold Spring Harbor Press, Cold Spring Harbor Laboratory, Cold Spring Harbor, New York or Current Protocols in Molecular Biology (1989) and supplements. The techniques involved in the transformation of E. coli cells used in the preferred practice of the invention as exemplified herein are well known in the art. The precise conditions under which the transformed E. coli cells are cultured is dependent on the nature of the E. coli host cell line and the expression or cloning vectors employed. For example, vectors which incorporate thermoinducible promoter-operator regions, such as the c1857 thermoinducible lambda-phage promoter-operator region, require a temperature shift from about 30 to about 40 degrees C. in the culture conditions so as to induce protein synthesis.

In the preferred embodiment of the invention, E. coli K12 RV308 cells are employed as host cells but numerous other cell lines are available such as, but not limited to, E coli K12 L201, L687, L693, L507, L640, L641, L695, L814 (E. coli B). The transformed host cells are then plated on appropriate media under the selective pressure of the antibiotic corresponding to the resistance gene present on the expression plasmid. The cultures are then incubated for a time and temperature appropriate to the host cell line employed.

Proteins which are expressed in high-level bacterial expression systems characteristically aggregate in granules or inclusion bodies which contain high levels of the overexpressed protein. Kreuger et al., in Protein Folding, Gierasch and King, eds., pgs 136-142 (1990), American Association for the Advancement of Science Publication No. 89-18S, Washington, D.C. Such protein aggregates must be solubilized to provide further purification and isolation of the desired protein product. Id. A variety of techniques using strongly denaturing solutions such as guanidinium-HCl and/or weakly denaturing solutions such as dithiothreitol (DTT) are used to solubilize the proteins. Gradual removal of the denaturing agents (often by dialysis) in a solution allows the denatured protein to assume its native conformation. The particular conditions for denaturation and folding are determined by the particular protein expression system and/or the protein in question.

Preferably, the present DNA sequences are expressed with a dipeptide leader sequence encoding Met-Arg or Met-Tyr as described in U.S. Patent No. 5,126,249, herein incorporated by reference. This approach facilitates the efficient expression of proteins and enables rapid conversion to the active protein form with Cathepsin C or other dipeptidylpeptidases. The purification of proteins is by techniques known in the art and includes reverse phase chromatography, affinity chromatography, and size exclusion.

The claimed proteins contain cysteine residues. Thus, a di-sulfide bond may be formed to stabilize the protein. For example, the present invention includes proteins of the SEQ ID NO: 1 wherein the Cys at position 96 of SEQ ID NO: 1 is crosslinked to Cys at position 146 of SEQ ID NO: 1 as well as those proteins without such di-sulfide bonds. In addition the proteins of the present invention may exist, particularly when formulated, as dimers, trimers, tetramers, and other multimers. Such multimers are included within the scope of the present invention.

### Example 5

The protein of SEQ ID NO: 7 with a Met Arg leader sequence was expressed in E.coli. Granules were solubilized in 8 M urea containing 5 mM cysteine. The protein was purified by anion exchange in 8 M urea containing 5 mM cysteine and folded by dilution into 8M urea (containing 5 mM cysteine, 0.1M TRIS at pH 9.0) and exhaustive dialysis against PBS. Following final purification of the proteins by size exclusion chromatography the proteins were concentrated to 3-3.5 mg/mL in PBS.

### Example 6

The protein of SEQ ID NO: 7 with a Met Arg leader sequence was expressed in E.coli, isolated and folded by techniques analogous to the previous Examples. The pH of the protein solution was reduced to pH 2.8. The Met Arg leader sequence was cleaved by the addition of 6-10 milliunits dDAP per mg of protein. The conversion reaction was allowed to proceed for 2-8 hours at room temperature. The progress of the reaction was monitored by high performance reversed phase chromatography. The reaction was terminated by adjusting the pH to 8 with NaOH. The des(Met-Arg) protein was further purified by cation exchange chromatography in the presence of 7-8 M urea and size exclusion chromatography in PBS. Following final purification of the proteins by size exclusion chromatography the proteins were concentrated to 3-3.5 mg/mL in PBS. The structure was confirmed by mass spectroscopy.

The present invention provides a method for treating a warm-blooded animal. The preferred use in such species is to regulate adipose tissue and thereby lower body weight. The method comprises administering to the animal an effective amount of protein in a dose between about .001 and 10 g/kg. A preferred dose is from about 0.01 to 10 g/kg of active compound. A typical daily dose for a mature species is from about 0.5 to 1000 mg. In practicing this method, proteins of SEQ ID NO: 1 can be administered in a single daily dose or in multiple doses per day. The treatment regime may require administration over extended periods of time. The amount per administered dose or the total amount administered will be determined by the physician and depend on severity of the disease, the age and general health of the patient and the tolerance of the patient to the compound.

The instant invention further provides formulations comprising proteins of SEQ ID NO: 1. The proteins, preferably in the form of a biologically acceptable salt, can be formulated for parenteral administration for the therapeutic or prophylactic treatment of obesity. For example, proteins of SEQ ID NO: 1 can be admixed with conventional pharmaceutical carriers and excipients. The compositions comprising claimed proteins contain from about 0.1 to 90% by weight of the active protein, preferably in a soluble form, and more generally from about 10 to 30%. Furthermore, the present proteins may be administered alone or in combination with other adipose regulating compounds. For intravenous (iv) use, the protein is administered in commonly used intravenous fluid(s) and administered by infusion. Such fluids, for example, physiological saline, Ringer's solution or 5% dextrose solution can be used. For intramuscular preparations, a sterile formulation, preferably a suitable soluble salt form of a protein of SEQ ID NO: 1, for example the hydrochloride salt, can be dissolved and administered in a pharmaceutical diluent such as pyrogen-free water (distilled), physiological saline or 5 % glucose solution. A suitable insoluble form of the compound may be prepared and administered as a suspension in an aqueous base or a pharmaceutically acceptable oil base, e.g. an ester of a long chain fatty acid such as ethyl oleate.

The ability of the present compounds to treat obesity and regulate adipose tissue is demonstrated *in vivo* as follows:

### Example 7

### Biological Testing

Parabiotic experiments suggest that a protein is released by peripheral adipose tissue and that the protein is able to control body weight gain in normal, as well as obese mice. Therefore, the most closely related biological test is to inject the test article by any of several routes of administration (i.v., s.c., i.m., i.p., or by minipump or cannula) and then to monitor food and water consumption, body weight gain, plasma chemistry or hormones (glucose, insulin, ACTH, corticosterone, GH, T4) over various time periods. Suitable test animals include normal mice (ICR, etc.) and obese mice *(ob/ob,* Avy/a, KK-Ay, tubby, fat). The *ob/ob* mouse model of obesity and diabetes is generally accepted in the art as being indicative of the obesity condition. Controls for non-specific effects for these injections are done using vehicle with or without the active agent of similar composition in the same animal monitoring the same parameters or the active agent itself in animals that are thought to lack the receptor (db/db mice, fa/fa or cp/cp rats). Proteins demonstrating activity in these models will demonstrate similar activity in other mammals.

Since the target tissue is expected to be the hypothalamus where food intake and lipogenic state are regulated, a similar model is to inject the test article directly into the brain (e.g. i.c.v. injection via lateral or third ventricles, or directly into specific hypothalamic nuclei (e.g. arcuate, paraventricular, perifornical nuclei). The same parameters as above could be measured, or the release of neurotransmitters that are known to regulate feeding or metabolism could be monitored (e.g. NPY, galanin, norepinephrine, dopamine, b-endorphin release).

Similar studies are accomplished *in vitro* using isolated hypothalamic tissue in a perifusion or tissue bath system. In this situation, the release of neurotransmitters or electrophysiological changes is monitored.

The ability of the proteins of the present invention to treat obesity in an OB/OB mouse is presented in the following table for a representative protein.

### Protein of Example 6

| Dose | Food Intake (g/mouse) | | | Food Intake (%control) | | | Body Weight Δ | | |
|---|---|---|---|---|---|---|---|---|---|
| µg | Day 1 | Day 2 | Day 3 | Day 1 | Day 2 | Day 3 | BWΔ1 | BWΔ2 | BWΔ3 |
| 0 | 6.4 | 5.9 | 6.7 | -- | -- | -- | 0.3 | 0.8 | 1.0 |
| 30 | 5.1 | 4.8 | 4.4 | 79.7 | 75.0 | 68.8 | -0.3 | -0.1 | -0.3 |
| 272 | 3.3 | 1.4 | 0.6 | 51.6 | 21.9 | 9.4 | -0.9 | -2.3 | -3.7 |

The proteins are active in at least one of the above biological tests and are anti-obesity agents. As such, they are useful in treating obesity and those conditions implicated by obesity. However, the proteins are not only useful as adipose regulating agents; one skilled in the art recognizes that the proteins are useful in the production of antibodies for diagnostic use and, as proteins, are useful as feed additives for animals. Furthermore, the compounds are useful for controlling weight for cosmetic purposes in warm-blooded animals. A cosmetic purpose seeks to control the weight of an animal to improve bodily appearance. Such cosmetic use forms part of the present invention. The claimed DNA sequences are useful for preparing protein compositions, for screening, and for preparing immunogens to raise antibodies for diagnostic use.

The principles, preferred embodiments and modes of operation of the present invention have been described in the foregoing specification. The invention which is intended to be protected herein, however, is not to be construed as limited to the particular forms disclosed, since they are to be regarded as illustrative rather than restrictive. Variations and changes may be made by those skilled in the art without departing from the spirit of the invention.

## Claims

1. A protein of the Formula I: 
(SEQ ID NO: 1)

Val Pro Ile Xaa Xaa Val Gln Asp Asp Thr Lys Thr Leu Ile Lys Thr
1               5                   10                  15

Ile Val Thr Arg Ile Xaa Asp Ile Ser His Xaa Xaa Ser Val Ser Ser
            20                  25                  30

Lys Gln Arg Val Thr Gly Leu Asp Phe Ile Pro Gly Leu His Pro Xaa
        35                  40                  45

Leu Ser Leu Ser Lys Met Asp Gln Thr Leu Ala Ile Tyr Gln Gln Ile
    50                  55                  60

Leu Thr Ser Leu Pro Ser Arg Asn Val Xaa Gln Ile Ser Asn Asp Leu
65                  70                  75                  80

Glu Asn Leu Arg Asp Leu Leu His Leu Leu Ala Xaa Ser Lys Ser Cys
                85                  90                  95

Pro Leu Pro Gln Xaa Arg Ala Leu Glu Xaa Leu Glu Ser Leu Gly Xaa
            100                 105                 110

Val Leu Glu Ala Ser Leu Tyr Ser Thr Glu Val Val Ala Leu Ser Arg
        115                 120                 125

Leu Gln Gly Xaa Leu Gln Asp Met Leu Arg Gln Leu Asp Leu Ser Pro
    130                 135                 140

Gly Cys
145
 wherein:
Xaa at position 4 is Trp or Cys;
Xaa at position 5 is Lys or Arg;
Xaa at position 22 is Ser or Asn;
Xaa at position 27 is Met or Thr;
Xaa at position 28 is Gln or absent;
Xaa at position 48 is Val or Leu;
Xaa at position 74 is Ile or Val;
Xaa at position 92 is Ser or Ala;
Xaa at position 101 is Ala or Val;
Xaa at position 106 is Thr or Ser;
Xaa at position 112 is Gly or Val; and
Xaa at position 132 is Ala or Ser;
or a pharmaceutically acceptable salt thereof.

2. The protein of Claim 1, which is SEQ ID NO:3, SEQ ID NO:5, or SEQ ID NO:7.

3. An isolated DNA molecule consisting of a nucleotide sequence that encodes the protein of Claim 1 or 2.

4. A recombinant DNA vector comprising a DNA molecule of Claim 3.

5. A recombinant host cell comprising a vector of Claim 4.

6. A process for producing a protein of Claim 1 or 2 from a precursor protein comprising the protein, which comprises:
(a) culturing a host cell of Claim 5;
(b) optionally processing the precursor protein to produce the protein of Claim 1 or 2; and
(c) isolating the protein.

7. A protein of Claim 1 or 2 for use as a pharmaceutical or veterinary agent.

8. A pharmaceutical formulation, which comprises a protein of Claim 1 or 2 together with one or more pharmaceutical acceptable diluents, carriers or excipients therefor.

9. A protein of Claim 1 or 2, which further comprises a leader sequence of the formula:
Met A₀ - ;
wherein A₀ is any amino acid except Pro.

10. A protein of Claim 9, wherein the leader sequence is Met Arg.
